# EUROPEAN PATENT SPECIFICATION

(11) **EP 0 623 321 B1**
(45) Date of publication and mention of the grant of the patent: **02.01.1997**
(21) Application number: 94201303.8
(22) Date of filing: 09.05.1994
(51) Int. Cl.: A61F 2/36

(54) **Femoral part of a hip joint prosthesis**
Femurteil einer Hüftgelenkprothese
Partie fémorale de prothèse de hanche

(30) Priority: 07.05.1993 DE 4315143
(43) Date of publication of application: 09.11.1994
(73) Proprietor: Bristol-Myers Squibb Company, New York, N.Y. 10154 (US)
(72) Inventor: Schug, Martin, D-23552 Lübeck (DE); Rischke, Burkhard, D-25462 Rellingen (DE)
(74) Representative: Mays, Julie

(56) References cited:
- EP-A- 0 181 586
- EP-A- 0 222 236
- EP-A- 0 238 860
- EP-A- 0 560 691
- FR-A- 2 538 242
- US-A- 3 064 645
- US-A- 5 007 931

## Description

The invention concerns a femoral part of a hip joint prosthesis to be anchored without cement, having a shaft with a narrow core and several longitudinal ribs extending outward from the core.

Such a femoral part is known, for example, from German Patent 28 39 092 A1, in which the shaft is straight and has several longitudinal ribs extending radially outward with constant thickness of the ribs, and which have self-cutting scraping teeth on the edges of the longitudinal ribs. Because of these properties this known femoral part can be driven into a prepared channel formed in the femur. In this case the channel has a diameter greater than that of the shaft core, dimensioned so that only the scraping teeth on the edges of the longitudinal ribs contact the bone, cutting out a groove for each longitudinal rib. The femoral prosthesis is surrounded by adjacent cancellous tissue only at the edges of the longitudinal ribs. The rest of the longitudinal ribs and the shaft core are free in the channel and are not supported by adjacent cancellous tissue. The primary stability of the femoral part, at least - that is, the stability of the femoral part immediately after its implantation - is not satisfactory for this known hip prosthesis.

From US-A-3064 645 which discloses the features of the preamble of claim 1, there is also known a femoral part of a hip implant having a shaft with a narrow shaft core and ribs extending radially out of the shaft core, the ribs tapering radially toward their edges and longitudinally toward the distal shaft end.

The objective of this invention, therefore, is to develop a femoral part of the type initially mentioned which has adequate primary and long-term stability following implantation, with minimum removal of bone tissue.

This objective is achieved for the femoral part of the type initially mentioned with the features of the characterizing part of claim 1. The longitudinal ribs are directed radially outward from the shaft core and are tapered both toward the outside and toward the distal end of the shaft.

In this way, with the longitudinal ribs directed radially outward in the form of a star, tapering both toward the outside and toward the distal end of the shaft, it is possible, with a sufficiently narrow shaft core, to drive the femoral part directly into the cancellous tissue of the femoral bone, with the part cutting its own channel, so that no socket channel need be previously formed in the cancellous tissue. When the femoral part is driven in, the cancellous tissue is displaced and compressed by the shaft core and the longitudinal ribs. Once driven into place, the shaft is completely surrounded by compressed cancellous tissue, and is held solidly in this compressed cancellous tissue by the pressure of that tissue. By this means a solid bond is attained between the shaft and the femur. In particular, the initial stability of the implant is increased.

The longitudinal ribs are provided in the lateral and medial regions as well as in the dorsal and ventral regions of the shaft core. The radial extent of all the longitudinal ribs decreases in the longitudinal direction from proximal to distal so that the outside contour of the shaft covered by the longitudinal ribs approximately matches the inside contour of the hard bone of the femur. Then the longitudinal ribs with their open edges extend nearly to the hard bone, thus producing a particularly firm bond between the implant and the bone, with the shaft being driven in until the edges of the longitudinal ribs are supported on the hard bone. In the embodiment of the invention the longitudinal ribs in the medial region taper off more rapidly from proximal to distal than do the lateral longitudinal ribs. Then the edges of the longitudinal ribs stop very near the cortex of the bone while the longitudinal axis of the shaft core coincides approximately with the longitudinal axis of the femur when the femoral part is implanted in the bone.

The radial extension of the longitudinal ribs in the proximal region of the shaft is preferably from about 0.2 to 1.2 times the maximum diameter of the shaft core. This dimensioning provides a balanced proportion between the implant volume and the remaining volume of bone tissue, so as to attain sufficient compression of the bone tissue and therefore a particularly solid bond between the implant and the bone.

Longitudinal ribs with perforations through which the remaining bone tissue can grow are particularly preferred. In order to increase the strength, it is preferable for the perforations in the longitudinal ribs to have at least partly an open-cell or open-pore structure through which the adjacent bone tissue grows, so that the attachment between the implant and the bone tissue is improved over a large area.

It is also particularly preferred for the surfaces of the longitudinal ribs to have at least partially open-cell or open-pore structure, and perforations can also be produced in the shaft core so that the adjacent bone tissue can grow into the surface structure and/or through the shaft core.

It is preferable for the longitudinal ribs to be tapered down toward the edges and rounded at their corners to prevent excessive linear stresses at the edges. Alternatively or additionally, wider supporting surfaces can be provided, at least in sections, in order to prevent excessive linear stresses at the longitudinal edges.

In one preferred embodiment of the invention the longitudinal ribs are provided only on the proximal portion of the shaft core. The distal part of the shaft core has no ribs and is smooth, or has a coated surface structure. The proximal end of the shaft has a collar which transforms into a neck to accept a prosthesis head. The collar and neck are a separate part which can be fastened removably to the shaft. Alternatively, the prosthesis head is also integral with the neck and collar. The neck part can be fastened to the shaft core by one or more screws or, alternatively, by a cone and collet connection.

Advantageous improvements of the invention are characterized by the features of the dependent claims.

In the following, embodiments of a femoral part according to the invention are explained in more detail by means of drawings.
They show:
- Figure 1.: A lateral view of the femoral part of a hip joint prosthesis with a separate neck part.
- Figure 2.: A cross-section along the line A-A in Figure 1.
- Figure 3.: A cross-section along the line B-B in Figure 1.
- Figure 4.: A femoral part according to Figure 1 implanted in a femur; and
- Figure 5.: Another embodiment of the femoral part without a neck part.

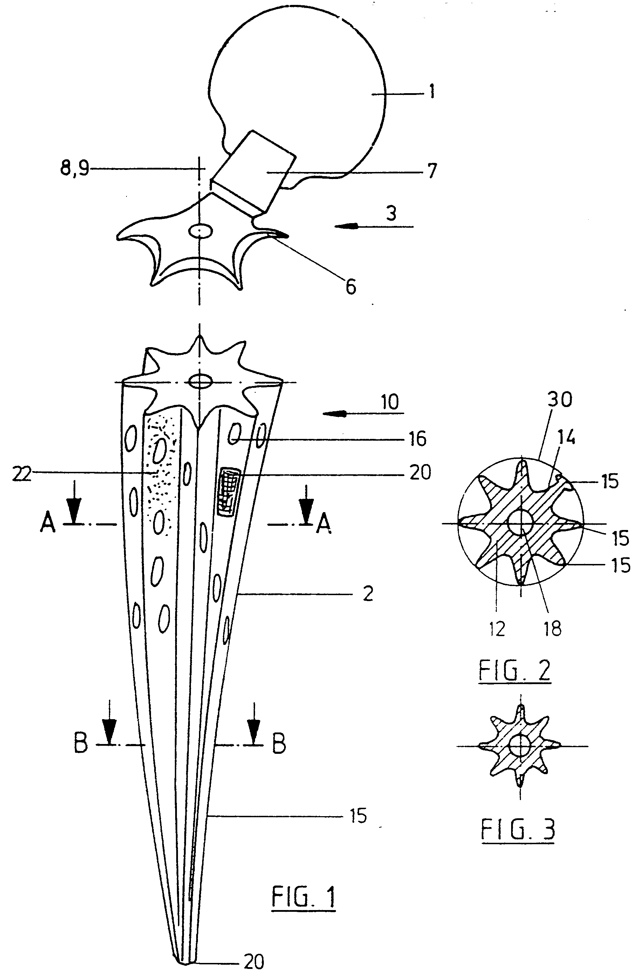

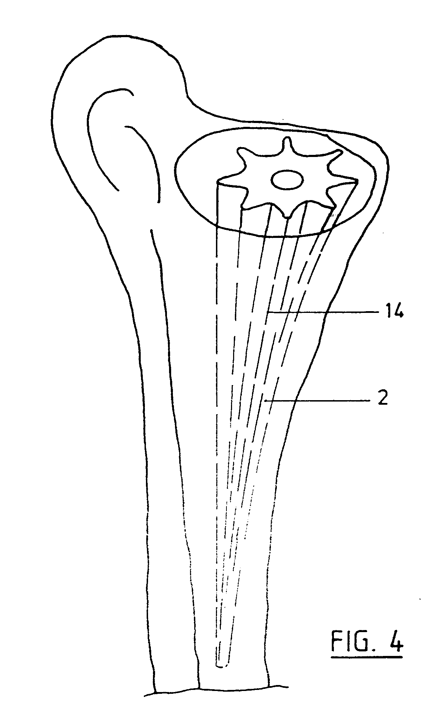

Figures 1 to 4 show a femoral part of a hip joint prosthesis which is intended to be implanted without cement in the upper part of a human femur. A shaft 2 has a narrow shaft core 12 of the prosthesis material, from which several longitudinal ribs 14 extend radially outward. The thickness of the longitudinal ribs diminishes radially outward to the edge 15. The thickness of the longitudinal ribs 14 also diminishes in the longitudinal direction toward the distal shaft end 20. At the proximal end of the shaft 2 there is a collar 6 which transforms into a neck, which is designed as a conical plug onto which a prosthesis head 1 with a matching conical hole can be mounted. The collar 6 and neck 7 form the neck part 3, which is fastened to the proximal end of the shaft 2 either by a screw connection 8, 9, 18 or by means of a cone and collet connection (not shown).

In the embodiment shown, longitudinal ribs 14 are arranged around the entire circumference of the shaft core 12. The longitudinal ribs in the medial region are longer than those in the lateral region. The radial extents of the longitudinal ribs are dimensioned so that the outer contour 30 of the shaft core with the longitudinal edges 15 will approximately match the inside contour of the medullary space of the proximal femur into which the shaft is to be implanted, except that the outside contour 30 of the shaft 2 is slightly smaller than the corresponding inside contour of the bone. In order to achieve this outside contour 30 the radial extension of the longitudinal ribs 14 decreases from proximal to distal. The shaft core 12, i. e., the internal solid cross section of the shaft 3, likewise narrows toward the distal shaft end 20. See also the shaft cross sections in Figures 2 and 3.

The radial extension of the longitudinal ribs 14 in the proximal region of the shaft 2 is from about 0.2 to 1.2 times the maximum diameter of the shaft core 12. According to the invention the radial extent of the longitudinal ribs in the lateral region is considerably less than in the medial region. The longitudinal ribs 14 also have a smaller peripheral spacing in the lateral region than in the medial region.

The longitudinal ribs are provided with perforations 16 which are at least partially provided with an open-cell or open-pore structure. The surfaces of the longitudinal ribs 14 are also provided at least partially with an open-cell or open-pore structure 29 into which adjacent cancellous tissue of the bone can grow. Perforations (not shown) can likewise be made in the shaft core 12 so that the cancellous tissue can grow through the shaft core.

The cross sections of the longitudinal ribs 14 taper toward their edges 15. The edges 15 are rounded in order to reduce linear stresses produced in the adjacent bone tissue at the edges. The shaft core has a central hole 18 from its proximal end. The upper end of the hold has an internal thread to accept screw 8 which can fasten the neck part 3 to the shaft 2.

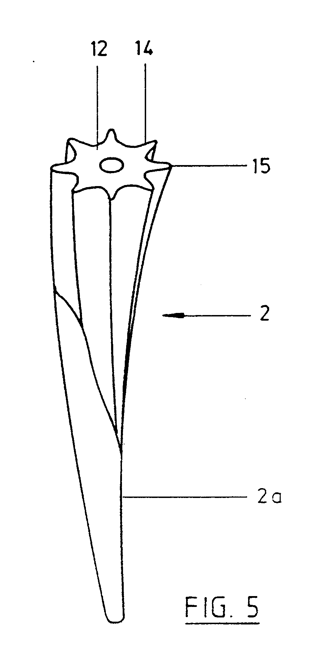

Figure 5 shows an alternative form of the shaft 2. Several longitudinal ribs 14 extend radially from the shaft core 12. As in the embodiment of Figures 1 to 4, these ribs taper down outward and distally. The longitudinal ribs 14 run from the proximal region of the shaft 2 to about the middle part of the shaft. Then they transform into an unribbed section 2a of the shaft 2. The medial longitudinal ribs 14 extend farther down toward the distal shaft end 20 than do the lateral longitudinal ribs. The unribbed shaft section 2a has a smooth or coated surface structure into which the cancellous tissue cannot grow. The longitudinal ribs 14 have penetrations and open-pore surface structure.

## Claims

1. Femoral part of a hip joint prosthesis for cementless fixation in a femur, having a shaft (2) with a narrow shaft core (12) and several longitudinal ribs extending radially outward from the shaft core (12), the longitudinal ribs (14) tapering continuously toward their edges(15) and longitudinally toward the distal shaft end(20) characterised in that the radial extent of the ribs (14) in the medial region of the shaft (2) decreases more sharply from the proximal end toward the distal end than that of the lateral longitudinal ribs (14).

2. Femoral part according to claim 1 characterised in that the radial extent of the longitudinal ribs (14) in the proximal region of the shaft (2) is between 0.2 and 1.2 times the maximum diameter of the shaft core (12).

3. Femoral part according to one of the preceding claims characterised in that the diameter of the shaft core (12) decreases from proximal to distal ends.

4. Femoral part according to one of the preceding claims characterised in that the proximal shaft core (12) has a central hole (18).

5. Femoral part according to one of the preceding claims characterised in that the longitudinal ribs (14) have perforations (16).

6. Femoral part according to claim 5 characterised in that the perforations (16) in the longitudinal ribs (14) at least have an open cell or open pore structure (20).

7. Femoral part according to one of the preceding claims characterised in that the surfaces of the longitudinal ribs (14) at least partially have an open-cell or open-pore structure (29).

8. Femoral part according to one of the preceding claims characterised in that the shaft core (12) is provided with perforations.

9. Femoral part according to claim 8 characterised in that the perforations of the shaft core (12) are placed in the distal region of the shaft core (12).

10. Femoral part according to one of the preceding claims characterised in that the longitudinal ribs (14) have widened supporting surfaces on their edges (15).

11. Femoral part according to one of the preceding claims characterised in that the edges (15) of the longitudinal ribs (14) have a rounded cross-section.

12. Femoral part according to one of the preceding claims characterised in that the longitudinal ribs (14) occur only in the proximal region of the shaft core (12).

13. Femoral part according to claim 12 characterised in that the unribbed region of the shaft (2) has a smooth or coated surface structure.

14. Femoral part according to one of the preceding claims characterised in that the proximal end of the shaft ends in a collar (6) which transforms into a neck (7) to accept a prosthesis head.

## Patentansprüche

1. Femurteil einer Hüftgelenkprothese zum zementlosen Befestigen in einem Oberschenkelknochen, der einen Schaft (2) mit einem sich verjüngenden Schaftkern (12) und mehrere radial aus dem Schaftkern (12) ragende Längsrippen aufweist, wobei sich die Längsrippen (14) zu ihren Kanten (15) und in Längsrichtung zu ihrem distalen Schaftende (20) hin verjüngen, dadurch gekennzeichnet, daß die radiale Ausdehnung der Rippen (14) in dem mittleren Bereich des Schafts (2) von dem proximalen Ende zu dem distalen Ende hin stärker abnimmt als die der seitlichen Längsrippen (14).

2. Femurteil nach Anspruch 1, dadurch gekennzeichnet, daß die radiale Ausdehnung der Längsrippen (14) in dem proximalen Bereich des Schafts (2) zwischen dem 0,2- und dem 1,2-fachen maximalen Durchmesser des Schaftkerns (12) liegt.

3. Femurteil nach einem der vorhergehenden Ansprüche, dadurch gekennzeichnet, daß der Durchmesser des Schaftkerns (12) vom proximalen zum distalen Ende hin abnimmt.

4. Femurteil nach einem der vorhergehenden Ansprüche, dadurch gekennzeichnet, daß der proximale Schaftkern (12) ein zentrales Loch (18) aufweist.

5. Femurteil nach einem der vorhergehenden Ansprüche, dadurch gekennzeichnet, daß die Längsrippen (14) Perforationen (16) aufweisen.

6. Femurteil nach Anspruch 5, dadurch qekennzeichnet, daß die Perforationen (16) in den Längsrippen (14) zumindest eine Struktur mit offenen Zellen oder offenen Poren (29) aufweisen.

7. Femurteil nach einem der vorhergehenden Ansprüche, dadurch gekennzeichnet, daß die Oberflächen der Längsrippen (14) zumindest teilweise eine Struktur mit offenen Zellen oder offenen Poren (29) aufweisen.

8. Femurteil nach einem der vorhergehenden Ansprüche, dadurch gekennzeichnet, daß der Schaftkern (12) mit Perforationen versehen ist.

9. Femurteil nach Anspruch 8, dadurch gekennzeichnet, daß die Perforationen des Schaftkerns (12) in dem distalen Bereich des Schaftkerns (12) angeordnet sind.

10. Femurteil nach einem der vorhergehenden Ansprüche, dadurch gekennzeichnet, daß die Längsrippen (14) an ihren Kanten (15) verbreiterte Stützflächen aufweisen.

11. Femurteil nach einem der vorhergehenden Ansprüche, dadurch gekennzeichnet, daß die Kanten (15) der Längsrippen (14) einen abgerundeten Querschnitt aufweisen.

12. Femurteil nach einem der vorhergehenden Ansprüche, dadurch gekennzeichnet, daß die Längsrippen (14) nur in dem proximalen Bereich des Schaftkerns (12) vorhanden sind.

13. Femurteil nach Anspruch 12, dadurch gekennzeichnet, daß der ungerippte Bereich des Schafts (2) eine glatte oder beschichtete Oberflächenstruktur aufweist.

14. Femurteil nach einem der vorhergehenden Ansprüche, dadurch gekennzeichnet, daß das proximale Ende des Schafts in einer Manschette (6) endet, die in einen Hals (7) übergeht, um einen Prothesenkopf aufzunehmen.

## Revendications

1. Partie fémorale d'une prothèse de hanche pour une fixation sans ciment dans un fémur, ayant un arbre (2) avec un noyau d'arbre étroit (12) et plusieurs nervures longitudinales s'étendant radialement vers l'extérieur à partir du noyau d'arbre (12), les nervures longitudinales (14) s'amincissant continuement vers leurs arêtes (15) et longitudinalement vers leur extrémité d'arbre distale (20), caractérisée en ce que l'extension radiale des nervures (14) dans la région médiale de l'arbre (2) diminue plus raidement à partir de l'extrémité proximale vers l'extrémité distale que celle des nervures longitudinales latérales (14).

2. Partie fémorale selon la revendication 1, caractérisée en ce que l'extension radiale des nervures longitudinales (14) dans la région proximale de l'arbre (2) est entre 0,2 et 1,2 fois le diamètre maximal du noyau d'arbre (12).

3. Partie fémorale selon l'une des revendications précédentes, caractérisée en ce que le diamètre du noyau d'arbre (12) diminue de l'extrémité proximale vers l'extrémité distale.

4. Partie fémorale selon l'une des revendications précédentes, caractérisée en ce que le noyau d'arbre proximal (12) comporte un trou central (18).

5. Partie fémorale selon l'une des revendications précédentes, caractérisée en ce que les nervures longitudinales (14) ont des perforations (16).

6. Partie fémorale selon la revendication 5, caractérisée en ce que les perforations (16) dans les nervures longitudinales (14) comportent au moins une structure à cellule ouverte ou à pore ouvert (20).

7. Partie fémorale selon l'une des revendications précédentes, caractérisée en ce que les surfaces des nervures longitudinales (14) comportent au moins partiellement une structure à cellule ouverte ou à pore ouvert (29).

8. Partie fémorale selon l'une des revendications précédentes, caractérisée en ce que le noyau d'arbre (12) est muni de perforations.

9. Partie fémorale selon la revendication 8, caractérisée en ce que les perforations du noyau d'arbre (12) sont placées dans la région distale du noyau d'arbre (12).

10. Partie fémorale selon l'une des revendications précédentes, caractérisée en ce que les nervures longitudinales (14) ont des surfaces de support élargies sur leurs-arêtes (15).

11. Partie fémorale selon l'une des revendications précédentes, caractérisée en ce que les arêtes (15) des nervures longitudinales (14) ont une section transversale arrondie.

12. Partie fémorale selon l'une des revendications précédentes, caractérisée en ce que les nervures longitudinales (14) sont présentes seulement dans la région proximale du noyau d'arbre (12).

13. Partie fémorale selon la revendication 12, caractérisée en ce que la région non nervurée de l'arbre (2) comporte une structure de surface lisse ou revêtue.

14. Partie fémorale selon l'une des revendications précédentes, caractérisée en ce que l'extrémité proximale de l'arbre se termine par un collier (6) qui se transforme en un col (7) pour accepter une tête prothétique.
